# EUROPEAN PATENT SPECIFICATION

(11) **EP 1 015 967 B1**
(45) Date of publication and mention of the grant of the patent: **24.04.2002**
(21) Application number: 98946035.7
(22) Date of filing: 11.09.1998
(51) Int. Cl.: G06F 9/44, G06F 17/30

(54) **METHOD AND SYSTEM FOR SYNCHRONIZED ACQUISITION, PROCESSING AND SHARING OF INSTRUMENTATION DATA AND FOR SYNCHRONIZED CONTROL IN A CLIENT-SERVER NETWORK**
VERFAHREN UND SYSTEM ZUR SYNCHRONISIERTEN ERFASSUNG, VERARBEITUNG UND ZUTEILUNG VON INSTRUMENTATIONSDATEN UND ZUR SYNCHRONISIERTEN STEUERUNG IN EINEM CLIENT-SERVER NETZWERK
PROCEDE ET SYSTEME POUR L'ACQUISITION, LE TRAITEMENT ET LE PARTAGE SYNCHRONISES DE DONNEES FOURNIES PAR DES INSTRUMENTS, ET POUR LA COMMANDE SYNCHRONISEE DANS UN RESEAU CLIENT-SERVEUR

(30) Priority: 12.09.1997 US 928201
(43) Date of publication of application: 05.07.2000
(73) Proprietor: Boston Scientific Limited, St. Michael, Barbados (BB)
(72) Inventor: GRUNWALD, Sorin, Santa Clara, CA 95050 (US); SO, Masserati, H., Fremont, CA 94539 (US); CHIN, Richard, Fremont, CA 95035 (US)
(74) Representative: Evens, Paul Jonathan
(86) International application number: US9818933
(87) International publication number: WO9913400

(56) References cited:
- WO-A-97/18636
- E. STEINFIELD: "Leveraging browsers as universal GUIs" ELECTRONIC ENGINEERING TIMES, no. 932, 16 December 1996, XP002076444
- WILLIAMS T: "TOOLS AND PROTOCOLS LINK EMBEDDED SYSTEMS OVER THE INTERNET" ELECTRONIC DESIGN, vol. 45, no. 17, 18 August 1997, page 91/92, 96, 98 XP000733504
- "DISK DRIVE WITH EMBEDDED HYPER-TEXT MARKUP LANGUAGE SERVER" IBM TECHNICAL DISCLOSURE BULLETIN, vol. 38, no. 12, 1 December 1995, page 479 XP000588211

## Description

### BACKGROUND OF THE INVENTION

This invention relates generally to instrumentation display and control in a client-server network environment, and particularly to a network implementation of a medical instrumentation cluster using a network browser paradigm. This invention affects all medical real-time imaging systems.

In the past, several types of real-time medical imaging systems have been used. These types of real-time medical imaging systems have certain structural features which lead to several functional problems.

Structurally, the different types of real-time medical imaging systems .differ in terms of their computer architecture. Most of the past systems use proprietary software to control proprietary hardware. The rest of the past systems use commercially available (off-the-shelf) hardware components, such as digital signal processing (DSP) boards and/or software components, and, operating systems, to a limited extent. No matter what components are used by past systems, prior to this invention, their overall hardware/software architecture remains proprietary and closed.

Generally, such traditional real-time medical imaging systems are based on a closed, monolithic architecture. Usually, these real-time medical imaging systems employ an embedded solution in which a central microprocessor (or a bank of microprocessors) is "embedded" in proprietary or off-the-shelf hardware. The hardware is controlled by "embedded" software with a proprietary or off-the-shelf real-time executive kernel (or basic component of an operating system), with most definitely proprietary applications to control the hardware. This requires intimate coupling of software to the target hardware. With this approach, the real-time medical imaging system has the advantage of being able to quickly execute real-time tasks by closely controlling the hardware with minimal software layers. However, this type of system's critical disadvantage is that the most important software and hardware components are proprietary and developed, in one shape or form, internally.

Consequently, these types of real-time medical imaging systems have several significant functional disadvantages. These systems pose problems to users in terms of (1) the making of modifications to the system's software, (2) compatibility among diverse computer software components developed by different parties, (3) the gathering, processing, and sharing of image data from multiple instruments in multiple locations to multiple users in multiple locations, and (4) the integration of new software components into the existing system software. First, modifications to the system software of one of these systems incurs a considerable penalty in terms of (1) speed of development, (2) risks of affecting the rest of the software, and (3) difficulty of incorporating the changes without drastically altering the original design. Second, compatibility is a problem if the system software application was developed by a different party. Third, as in our design, an integrated approach to acquire or process data and to transfer the data across the network is difficult with such a monolithic design. Consequently, the sharing of instrumentation data is hampered by these systems because, typically, a user using one of these systems can monitor only one instrument at a time and can monitor that instrument from only one location. Finally, as new developments occur in the system's software, integration of these new software developments is made difficult by two factors. First, integration requires the integrator to have access to either the source code or a linkable library file that is compatible with the system's original software platform in terms of the language, hardware platform, etc. Second, even if the source code or the library were available, integration is still made difficult because it requires that the developer understand the internal design of the new software component in order to effectively use it within the system software.

The Internet provides users with a means of communicating information, ideas, and messages and with a means to design reusable software written in a highly standardized computer language. The Internet is a global computer network, consisting of Web clients and Web servers, which allows a user at a Web client to access information through the use of an Internet Web browser running on the Web client. Through the Web browser, the user accesses a particular Web server through an Internet link.

By connecting to the Internet, the user can retrieve information from a multitude of Web sites, where each Web site is located at a Web server. A Web site is a series of screen displays, Web pages, consisting of text, pictorial, and other information about a particular subject, organization, or company. A particular Web site can be retrieved from the Internet by establishing an Internet link to the Web site. In order to establish an Internet link to the Web site, the user would specify the Web site's Internet uniform resource locator (hereinafter URL) address to the Web browser or would click on a hypertext link on a Web page, with this hypertext link including the Web site's Internet URL address.

The Internet provides a means to design and implement reusable software comporients developed in a highly standardized environment, such as Java. Applets and/or ActiveX controls. Both Java Applets and ActiveX controls provide for modular, reusable code development. Such code can be easily transferred between systems in a network environment, such as the Internet. When a Web browser interprets a Web page which includes Java Applets or ActiveX controls, the Web browser retrieves a software object developed as a Java Applet or as an ActiveX control from a Web server on the computer network, such as the Internet. After retrieving this software object, the Web client, which is running the Web browser, makes use of the down-loaded software objects by running them on the Web client's system within the current Web page.

### SUMMARY OF THE INVENTION

According to the invention, a software/hardware architecture is provided which is based on an interactive graphical network model and which performs synchronized acquisition, processing and sharing of instrumentation data from at least one remote instrument coupled to one server and which synchronizes the control of devices including said instruments in a client-server network, with the client-server network comprising at least one client, at least one server, and a communications medium connecting each client to each server, whereby the architecture permits the performance of a series of synchronized functions. In a specific embodiment, responsive to input from a user at the client, the architecture transmits, from at least one of the servers to the client, a selected software control object associated with a selected instrument. Second, the software/hardware architecture displays a graphical representation of a functionality of the selected software control object on a display device of the client. Third, the software/hardware architecture, responsive to input from the user to the selected software control object. acquires instrumentation data from the selected instrument. Fourth, responsive to input from the user to the selected software control object, the software/hardware architecture stores the instrumentation data in a designated computer storage medium. Fifth, responsive to input from the user to a selected device driver associated with the selected instrument via the selected software control object, the software/hardware architecture sends the instrumentation data to the display device of the client for presentation to the user. Sixth, the software/hardware architecture, responsive to input from the user to the selected software control object, presents a graphical representation of the instrumentation data on the display device of the client to the user. Seventh, the software/hardware architecture manages the selected software control object at least for controlling a defined behavior of the selected software control object. Finally, the software/hardware architecture interfaces the selected software control object to the selected instrument via the client-server network and a device driver manager in each one of the servers.

Key components of the invention include the synchronization of the acquisition of the instrumentation data from the data acquisition elements and the synchronization of the presentation of this instrumentation data, such that the data acquisition tasks and the device control tasks are separated from the user interface tasks and data display tasks. In order to synchronize the acquisition of the instrumentation data from the data acquisition elements, the software/hardware architecture (the device driver manager in'the server) controls a timing sequence of the software/hardware architecture's interfacing (each device driver's timing sequence) of the selected software control object to the selected instrument via the client-server network. In order to synchronize the display of the instrumentation data on the display device of the client to the user, the software/hardware architecture (the software control object at the client) controls a timing sequence of the software/hardware architecture's presenting of the instrumentation data on the display device of the client to the user. In addition, the software/hardware architecture (the software control object manager at the server) manages the selected software control object by at least controlling the behavior of the selected software control object. In these three ways (the device driver manager in said server, the software control object in the client, and the software control object manager in the server), the software/hardware architecture additionally separates the data acquisition tasks and the device control tasks, tasks performed at the server, from the user interface tasks and display tasks, tasks performed at the client.

The invention will be better understood by reference to the following detailed description in connection with the following drawings.

### BRIEF DESCRIPTION OF THE DRAWINGS

Fig. 1 is a block diagram of a client-server network for data acquisition and control, according to the present invention.

Fig. 2 is a block diagram of the client-side system of the present invention, illustrating user interface elements and instrumentation data display elements.

Fig. 3 is a block diagram of the server-side system of the present invention, illustrating data acquisition elements and control interface elements.

Fig. 4 is a high-level flow chart illustrating the operation of the present invention between a client and a server.

Fig. 5 illustrates a system block diagram of a typical computer system used to execute the software of a client and a server in an embodiment of the present invention.

Fig. 6 is a block diagram of the Internet to which a client and a server, according to the present invention, can attach.

Fig. 7 is a timing diagram illustrating data acquisition timing according to the invention.

Fig. 8 illustrates a typical control object and data view according to the invention.

### DESCRIPTION OF SPECIFIC EMBODIMENTS

The invention is explained in the context of applications of medical instrumentation in a client-server networked environment, such as an Internet/intranet environment. However, this invention is applicable to analogous fields requiring real-time and/or quasi-real-time data acquisition and control. Therefore, the description of the embodiment that follows is for purposes of illustration and not limitation.

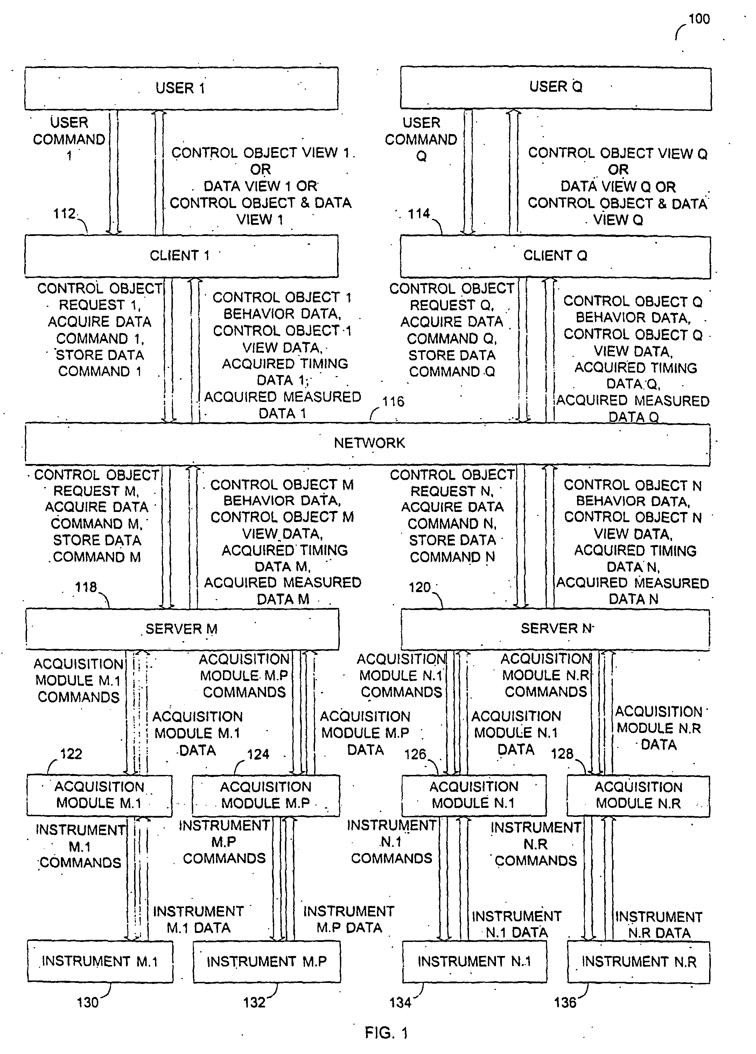

Fig. 1 is a block diagram of a client-server network for data acquisition and control, according to the present invention. Referring to Fig. 1, there is shown a system 100 comprising illustrative clients 112, 114 coupled via network 116 to a plurality of servers 118, 120, with each server 118, 120 being respectively connected to a plurality of acquisition modules 122, 124 and 126, 128, and, with each acquisition module 122, 124, 126, 128 being respectively connected to an instrument 130, 132, 134, 136.

A user at client 112, 114 sends a user command to client 112, 114. Depending upon the type of user command inputted, client 112, 114 generates and sends a software control object (hereinafter control object) request, an acquire data command, or a store data command via network 116 to server 118, 120. Also depending upon the type of user command inputted to client 112, 114, server 118, 120 respectively generates and respectively sends control object behavior data and control object view data to client 112, 114 via network 116, acquisition module commands to acquisition module 122, 124 and 126, 128, and/or acquired timing data and acquired measured data to client 112, 114 via network 116.

Acquisition module 122, 124, 126, 128 respectively sends instrument commands to instrument 130, 132, 134, 136. Instrument 130, 132, 134, 136 respectively generates and respectively sends instrument data to acquisition module 122, 124, 126, 128. Acquisition module 122, 124 and 126, 128 respectively generates and respectively sends acquisition module data to server 118, 120. Responsive to a command from a user, server, 118, 120 respectively generates and respectively sends control object behavior data, control object view data, acquired timing data, and acquired measured data via network 116 to client 112, 114. Client 112, 114 can display a control object view, a data view, or a control object and data view to the user on a display of the client.

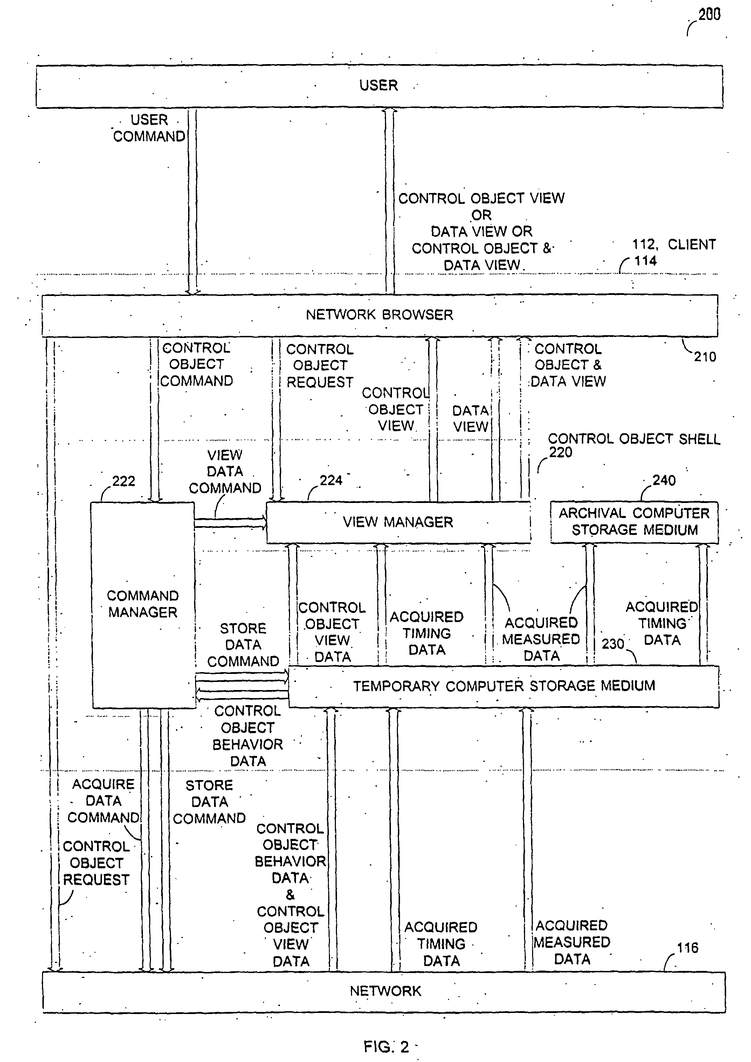

Fig. 2 is a block diagram of the client-side system of the present invention, illustrating user interface elements and instrumentation data display elements. Referring to Fig. 2, there is shown a system 200 comprising illustrative client 112, 114, connected to network 116. Client 112, 114 comprises network browser 210, control object shell 220, temporary computer storage medium 230, and archival computer storage medium 240. Control object shell 220 comprises command manager 222 and view manager 224.

In an embodiment of the present invention, a control object comprises a software object responsive to at least one input command to generate and send at least one output command based on a defined behavior of the software object. In addition, a control object is responsive to at least one input command to generate and send at least one set of data. A control object is associated with a particular instrument 130, 132, 134, 136. In an embodiment of the present invention, each control object is a software object which is accessed by Java Applets or ActiveX controls within a Web page.

A user inputs a user command into network browser 210 running on client 112, 114. Network browser 210 receives the user command and generates and sends either a control object request or a control object command. Depending upon the type of user command received, network browser 210 sends a control object request to server 118, 120 via network 116 and to view manager 224. Alternatively, network browser 210 sends a control object command to command manager 222.

If network browser 210 sends a control object request to server 118, 120 via network 116, temporary computer storage medium 230 receives control object behavior data and control object view data from server 118, 120 via network 116. In an embodiment of the present invention, a control object request would be generated by network browser 210 in response to a user's having selected a particular control object in order to control its associated instrument 130, 132, 134, 136. In addition, in an embodiment of the present invention, temporary computer storage medium 230 receives an electronic copy of a user interface module when temporary computer storage medium 230 receives control object behavior data and control object view data. Command manager 222 subsequently retrieves the control object behavior data from temporary computer storage medium 230.

View manager 224 also receives the control object request from network browser 210. View manager 224 subsequently retrieves the selected control object view data from temporary computer storage medium 230, and generates and sends a control object view to network browser 210. Network browser 210, thereafter, displays the control object view to the user on a display of the client. In an embodiment of the present invention, the displayed control object view is a graphical representation of a functionality of the selected control object.

In an embodiment of the present invention, network browser 210 is optimized for presenting the graphical representation of the functionality of the selected control object on the display device of client 112, 114.

In the present invention, the control object is responsive to control object commands, as follows. If command manager 210 receives a control object command from network browser 210, command manager 210 performs one of several functions. If the control object command corresponds to an acquire data command, command manager 222 sends an acquire data command to server 118, 120 via network 116, and, temporary computer. storage medium 230 receives acquired timing data and acquired measured data from server 118, 120 via network 116. If the control object command corresponds to a store data command, command manager 222 sends a store data command either to server 118, 120 via network 116 or to temporary computer storage medium 230. If, according to the control object command, command manager 222 sends the store data command to temporary computer storage medium 230, temporary computer storage medium 230 sends acquired measured data and acquired timing data to archival computer storage medium 240 for storage. Alternately, command manger 222 sends the store data command to server 118, 120 via network 116, and, server 118, 120 reacts accordingly, as described later in this description.

The present invention can provide several different views to the user. In addition to the aforementioned control object view, the present invention can provide a data view or a control object and data view. If command manager 222 sends to view manager 224 a view data command which indicates a request for a data view, view manager 224 retrieves the acquired timing data and the acquired measured data from temporary computer storage medium 230, and, subsequently, generates and sends a data view, comprising a graphical representation of the acquired measured data synchronized with the acquired timing data, to network browser 210. Network browser 210, thereafter, displays the data view to the user on the display of the client. Alternately, if command manager 222 sends to view manager 224 a view command which indicates a request for a control object and data view, view manager 224 retrieves the acquired timing data, the acquired measured data, and the control object view data from temporary computer storage medium 230, and, subsequently, generates and sends a control object and data view, comprising a graphical representation of the acquired measured data synchronized with the acquired timing data and a graphical, representation of a functionality of the selected control object, to network browser 210. Network browser 210, thereafter, displays the control object and data view to the user on the display of the client. By synchronizing the acquired measured data with the acquired timing data, view manager 224 effectively controls the timing sequence of the presentation of the graphical representation of the acquired timing data by network browser 210.

In an embodiment of the present invention, network browser 210 is optimized for presenting the graphical representation corresponding to the instrumentation data on the display device of client 112, 114.

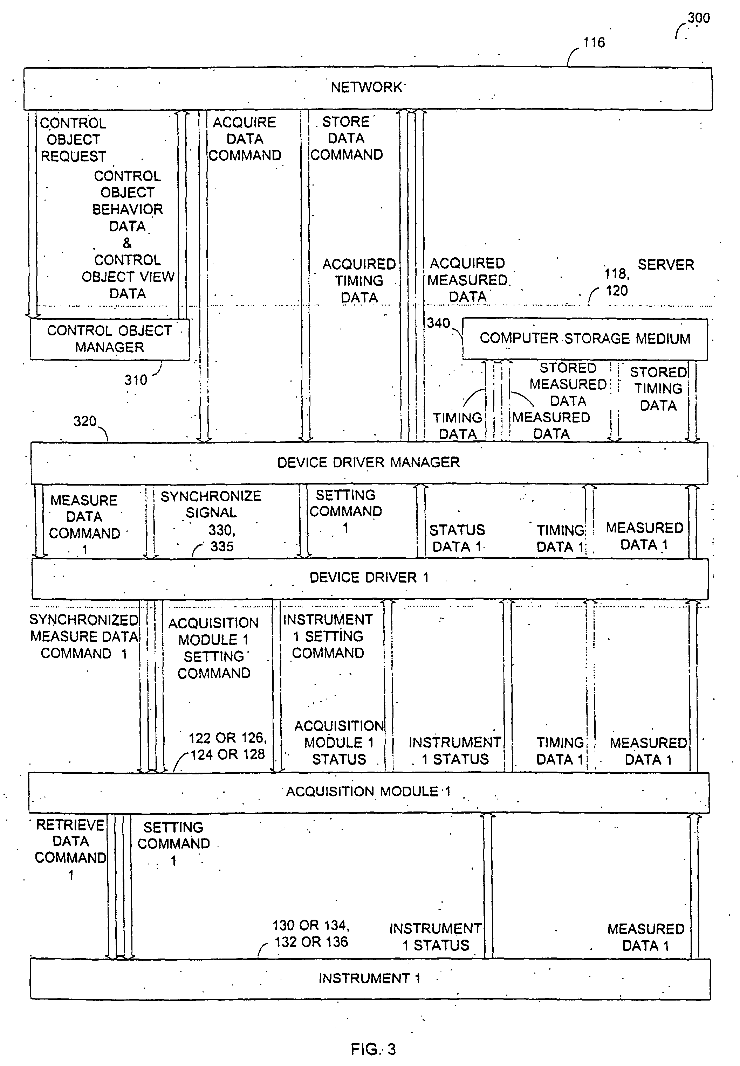

Fig. 3 is a block diagram of the server-side system of the present invention, illustrating data acquisition elements and control interface elements. Referring to Fig. 3, there is shown a system 300 comprising an illustrative server 118, 120 being respectively connected to network 116 and to at least one acquisition module 122 or 126, 124 or 128, with this acquisition module 122 or 126, 124 or 128 being respectively connected to instrument 130 or 134, 132 or 136. The server comprises control object manager 310, device driver manager 320, at least one device driver 330, 335, and computer storage medium 340.

When control object manager 310 receives a control object request from client 112, 114 via network 116, control object manager 310 generates and sends control object behavior data and control object view data to client 112, 114 via network 116. The control object manager manages the behavior of the control object that has been requested by defining at least one characteristic of the control object in terms of control object behavior data, by creating the control object when the control object is requested, and by deleting the control object when the control object is no longer needed.

When device driver manager 320 receives from client 112, 114 via network 116 an acquire data command which indicates a request to acquire data directly from instrument 130 or 134, 132 or 136, device driver manager 320 proceeds to generate and send a measure data command, a synchronize signal, and a setting command to device driver 330, 335. Device Driver 330, 335 respectively generates and respectively sends a synchronized measure data command, an acquisition module setting command, and an instrument setting command to acquisition module 122 or 126, 124 or 128. Acquisition module 122 or 126, 124 or 128, respectively generates and sends a retrieve data command and a setting command to instrument 130 or 134, 132 or 136.

Thereafter, instrument 130 or 134, 132 or 136, respectively generates and sends an instrument status and measured data to acquisition module 122 or 126, 124 or 128. Acquisition module 122 or 126, 124 or 128 respectively generates and sends an acquisition module status and timing data, and, respectively sends the instrument status and the measured data to device driver 330, 335. From this data, device driver 330, 335 respectively generates and sends status data, and, respectively sends the timing data and the measured data to device driver manager 320. Device driver manager 320 generates and sends acquired timing data and acquired measured data to client 112, 114 via network 116.

When device driver manager 320 receives from network 116 an acquire data command which indicates a request for stored data, device driver manager 320 proceeds to retrieve stored timing data and stored measured data, from computer storage medium 340. Thereafter, device driver manager 320 generates and sends acquired timing data and acquired measured data to client 112, 114 via network 116.

When device driver manager 320 receives a store data command from network 116, device driver manager 320 proceeds to generate and store timing data and measured data in computer storage medium 340.

Device drive manager 320, device driver 330, 335, and acquisition module 122 or 126, 124 or 128 accomplish the interfacing of a particular control object to a particular instrument 130 or 134, 132 or 136. Acquisition module 122 or 126, 124 or 128 responds to at least one real-time event in instrument 130 or 134, 132 or 136 via the instrument status sent from instrument 130 or 134, 132 or 136 and by issuing the setting command to instrument 130 or 134, 132 or 136. Device driver 330, 335 responds to at least one real-time event in instrument 130 or 134, 132 or 136 via the instrument status and acquisition module status sent from acquisition module 122 or 126, 124 or 128 and by issuing the acquisition module setting command and the instrument setting command to acquisition module 122 or 126, 124 or 128. Device Driver Manager 320 responds to at least one real-time event in instrument 130 or 134, 132 or 136 via the status sent from device driver 330, 335 and by issuing the setting command to device driver 330, 335.

Device driver manager 320 controls the timing sequence of the interfacing of a particular control object to a particular instrument 130 or 134, 132 or 136 by issuing the synchronize signal to device driver 330, 335, which in turn generates and sends the synchronized measure data command to acquisition module 122 or 126, 124 or 128.

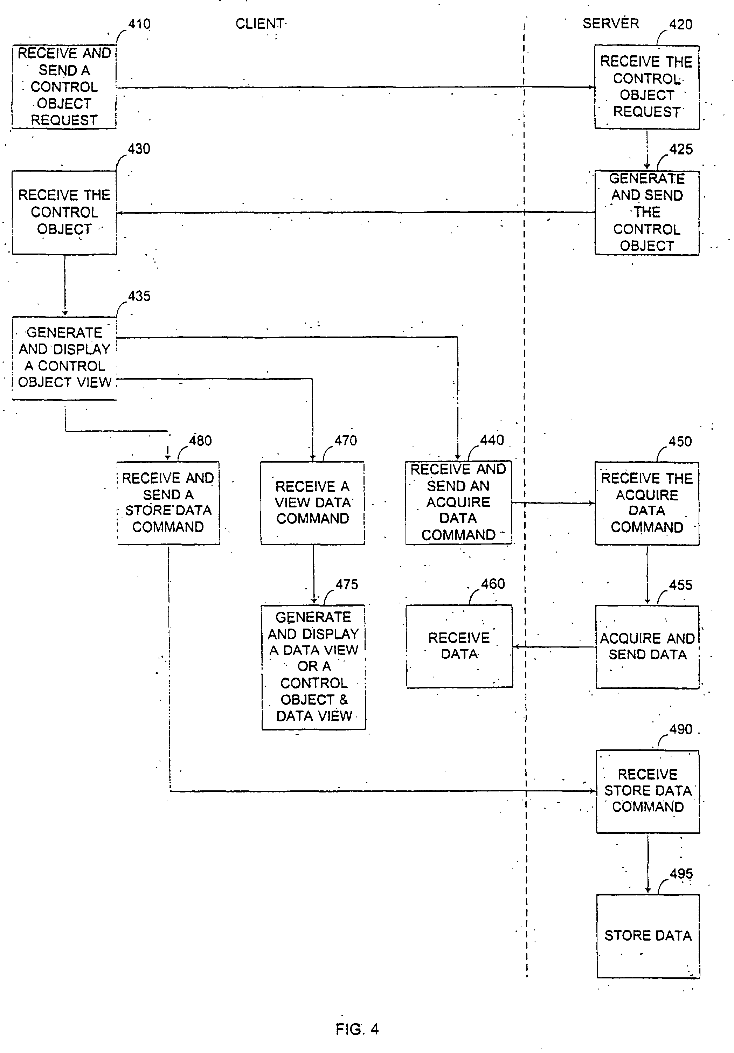

Fig. 4 is a high-level flow chart illustrating the operation of the present invention between a client and a server. First, at step 410, the client receives a control object request from the user and, subsequently, sends the control object request to the server via a network. Next, at step 420, the server receives the control object request. At step 425, the server generates and sends the requested control object to the client. At step 430, the client receives the control object. Next, at step 435, the client generates and displays a control object view.

At step 440, the client receives an acquire data command from the user, and, subsequently, sends the acquire data command to the server. At step 450, the server receives the acquire data command. At step 455, the server acquires data, both acquired measured data and acquired timing data, from an instrument via a corresponding acquisition module, and, subsequently, sends this data to the client. At step 460, the client receives this data.

At step 470, the client receives a view data command. Next, at step 475, the client generates and displays a data view or a control object and data view on the display of the client.

At step 480, the client receives a store data command, and, subsequently, sends the store data command to the server or stores the data within itself in an archival computer storage medium. Next, at step 490, the server receives the store data command. At step 495, the server stores the data in a computer storage medium.

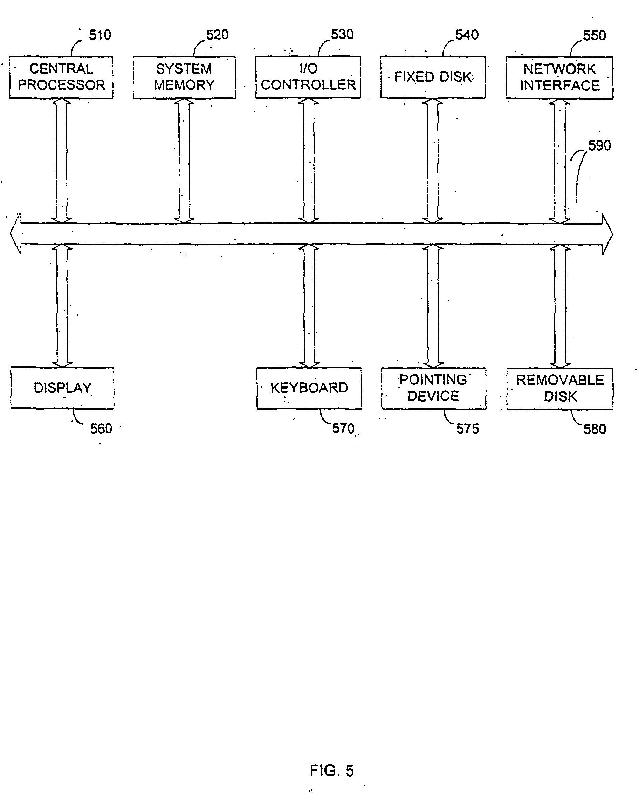

Fig. 5 illustrates a system block diagram of a typical computer system 500 used to execute the software of client 112, 114 and server 118, 120 in an embodiment of the present invention. Computer system 500 includes subsystems. such as a central processor 510, a system memory 520, an I/O control object 530, a fixed disk 540, and a network interface 550. Computer system 500 may also include a display 560, a keyboard 570, a pointing device 575, and a removable disk 580. Other computer systems suitable for use with the present invention may include additional or fewer subsystems. For example, another computer system could include more than one processor 510 (i.e., a multi-processor system) or a cache memory as part of system memory 520.

Arrows such as 590 represent the system bus architecture of computer system 500. However, these arrows are illustrative of any interconnection scheme serving to link the subsystems. For example, a local bus could be utilized to connect the central processor to the system memory and display adapter. Computer system 500 is but an example of a computer system suitable for use with the present invention. Other configurations of subsystems suitable for use with the present invention will be readily apparent to one of ordinary skill in the art, especially in the field of parallel processing.

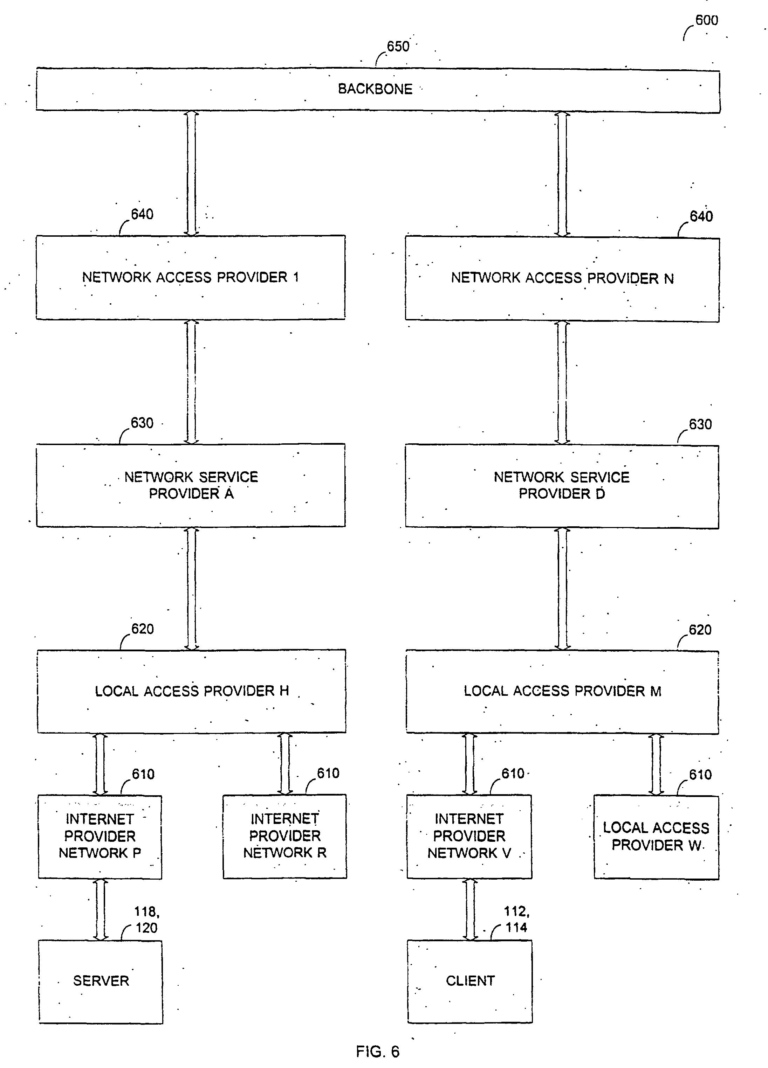

Fig. 6 is a block diagram of the Internet/intranet 600 to which client 112, 114 and server 118, 120, according to the present invention, can attach.

In the example shown, client 112, 114 is attached to an Internet provider network 610, and, server 118, 120 is attached to an Internet provider network 610. A number of Internet provider networks 610 provide connection to local access providers 620, which then provide access to network service providers 630. The network service providers 630 then connect to network access providers 640, which, in turn, are connected to the backbone service 650. It should be understood that other configurations of the Infernet or other networks could be used with the present invention.

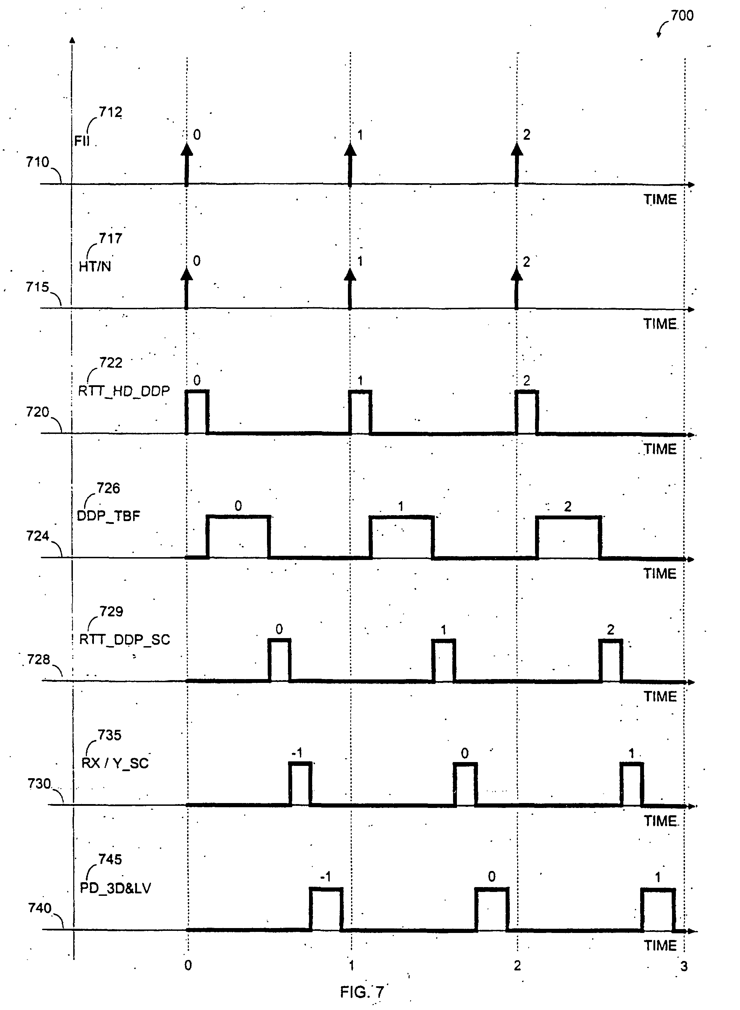

Fig. 7 illustrates timing diagram 700 depicting a typical data acquisition timing sequence according to the invention. Data trace 710 illustrates the value of frame index interrupt signal (FII) 712 over time. Data trace 715 illustrates the value of hardware toggle/notify signal (HT/N) 717 over time. Data trace 720 illustrates the value of R-Theta (hereinafter RT) transfer from hard disk (hereinafter HD) to the data dependent processing module (hereinafter DDP) input buffer signal (RTT_HD_DDP) 722 over time. Data trace 724 illustrates the value of DDP table based filtering signal (DDP_TBF) 726 over time. Data trace 728 illustrates the value of RT transfer from the DDP output buffer to the scan converter module (hereinafter SC) input buffer signal (RTT_DDP_SC) 729 over time. Data trace 730 illustrates the value of read X/Y from the SC output buffer for three dimensional volume construction and. rendering (hereinafter 3D) and long-view representation (hereinafter LV) signal (RX/Y_SC) 735 over time. Data trace 740 illustrates the value of process data for 3D and LV signal (PD_3D&LV) 745 over time.

In an embodiment of the present invention, FII 712 is an instrument status signal generated by and sent from instrument 130 or 134, 132 or 136. In another embodiment of the present invention, FII 712 is an instrument status signal sent from instrument 130 or 134, 132 or 136 via acquisition module 122 or 126, 124 or 128. In an embodiment of the present invention, HT/N 717 is an instrument status signal generated by and sent from instrument 130 or 134, 132 or 136. In another embodiment of the present invention, HT/N 717 is an instrument status signal sent from instrument 130 or 134, 132 or 136 via acquisition module 122 or 126, 124 or 128. In an embodiment of the present invention, RTT_HD_DDP 722 is a signal generated and used within acquisition module 122 or 126, 124 or 128. In an embodiment of the present invention, DDP_TBF 726 is a signal generated and used within acquisition module 122 or 126, 124 or 128. In an embodiment of the present invention, RTT_DDP_SC 729 is a signal generated and used within acquisition module 122 or 126, 124 or 128. In an embodiment of the present invention, read X/Y signal 735 is a signal generated and used within device driver 330, 335. In another embodiment of the present invention, RX/Y_SC 735 is a signal generated and used within device driver manager 320. In another embodiment of the present invention, RX/Y_SC 735 is a signal generated and used within view manager 224. In an embodiment of the present invention, process data signal 745 is a signal generated and used within device driver 330, 335. In another embodiment of the present invention, PD_3D&LV 745 is a signal generated and used within device driver manager 320. In another embodiment of the present invention, PD_3D&LV 745 is a signal generated and used within view manager 224.

In an embodiment of the present invention, each set of these signals represents a sequence of operations which can be performed by a single or multiple processors concurrently. In another embodiment of the present invention, several such sequences or parts of such a sequence, can be implemented in a parallel architecture comprising several processors executing parts of different sequences concurrently.

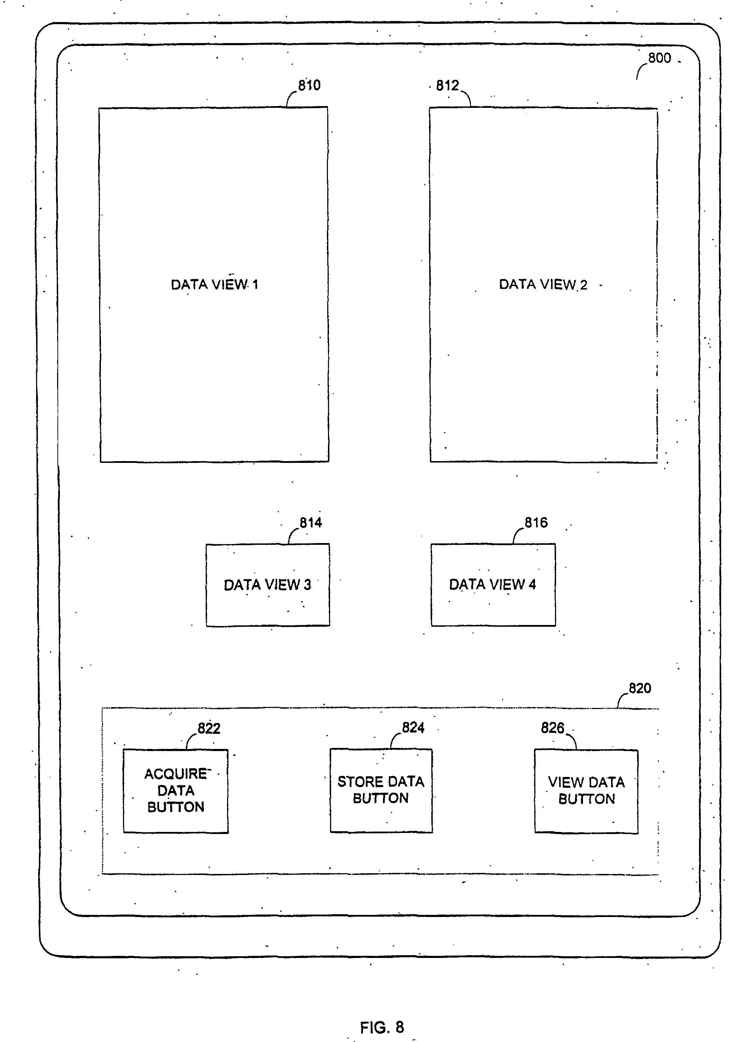

Fig. 8 illustrates a typical control object and data view 800 according to the invention. First data view 810 depicts one view of acquired measured data. Second data view 812 depicts a second view of acquired measured data. Third data view 814 depicts a third view of acquired measured data. In an embodiment of the present invention, third data view 814 is a smaller version of first data view 810. Fourth data view 816 depicts a fourth data view of acquired measured data. In an embodiment of the present invention, fourth data view 816 is a smaller version of second data view 812. Control object view 820 depicts and provides clickable buttons, such as acquire data button 822, store data button 824, and view data button 826, displayed on the client's display device and usable via the keyboard 570 or pointing device 575 of the client 112, 114. In an embodiment of the present invention, a user could send a control object command to acquire data by selecting acquire data button 822 with keyboard 570 or pointing device 575. In an embodiment of the present invention, a user could send a control object command to store data by selecting store data button 824 with keyboard 570 or pointing device 575. In an embodiment of the present invention, a user could send a control object command to view data by selecting view data button 826 with keyboard 570 or pointing device 575.

The invention has been explained with reference to a specific embodiment. Other embodiments will be apparent to those of ordinary skill in the art. It is therefore not intended that this invention be limited, except as indicated by the appended claims.

## Claims

1. A method for real-time synchronized processing of instrument (130,132,134,136) data comprising the steps of:
acquiring a plurality of frames of data from an instrument; and
for each such frame, sequentially performing a plurality of primary processing steps on the data of such frame according to a predetermined timing sequence that defines a start time and an end time for each primary processing step,
wherein the start time of each such primary processing step occurs substantially immediately after the end time of a preceding primary processing step.

2. The method according to claim 1, wherein each frame of data represents a visual image.

3. The method according to claim 2, wherein the plurality of primary processing steps includes:
storing such frame of data on a storage device (230);
filtering the stored data with a time filter; and
managing memory of the storage device (230) to prepare the filtered data for display of the visual image.

4. The method according to any one of claims 1 to 3, wherein the predetermined timing sequence further defines a start time and an end time for a secondary processing step, wherein the start time for the secondary processing step occurs substantially immediately after the end time of one of the primary processing steps, and
wherein the method further comprises performing the secondary processing step according to the predetermined timing sequence.

5. The method according to claim 4, wherein the start time for the secondary processing step occurs substantially immediately after the end time of a final primary processing step for such frame.

6. The method according to claim 4 or claim 5, wherein the secondary processing step is performed on a predecessor frame of data to such frame on which the plurality of first processing steps is performed.

7. The method according to any one of claims 4 to 6, wherein the secondary processing step includes displaying the visual image represented by the filtered data of a predecessor frame on a display device (560).

8. The method according to any one of claims 4 to 7, wherein the secondary processing step further includes converting the filtered data of the predecessor frame from a first coordinate system to a second coordinate system.

9. The method according to any one of the preceding claims, wherein the frames are acquired at regularly spaced time intervals.

10. The method according to any one of the preceding claims, wherein the step of sequentially performing a plurality of first processing steps is initiated by a frame index interrupt signal.

11. The method according to claim 1 comprising the steps of:
acquiring a plurality of frames of the image data from an instrument (130,132,134,136) at regularly spaced time intervals; and
for each such frame, in response to a frame index interrupt signal and according to a predetermined timing sequence:
storing the frame of image data on a storage device (230);
substantially immediately thereafter, filtering the stored data with a time filter;
substantially immediately thereafter, managing memory of the storage device (230) to prepare the filtered data for display;
substantially immediately thereafter, converting the filtered data of a predecessor frame from a first coordinate system to a second coordinate system; and
substantially immediately thereafter, displaying an image represented by the converted data of the predecessor frame on a display device (560).

12. An apparatus (100) for real-time synchronized processing of data from an instrument (130,132,134,136) comprising:
an acquisition module (122,124,126,128) configured to acquire a plurality of frames of data from the instrument; and
a processing module (220) configured to sequentially perform a plurality of primary processing steps on each such frame of data acquired by the acquisition module (122,124,126,128) according to a predetermined timing sequence that defines a start time and an end time for each primary processing step,
wherein the start time of each such primary processing step occurs substantially immediately after the end time of a preceding primary processing step.

13. The apparatus (100) according to claim 12, wherein each frame of data represents a visual image.

14. The apparatus (100) according to claim 13, further comprising a storage device (230) accessible by the processing module (220), and wherein the plurality of primary processing steps includes:
storing such frame of data on the storage device (230);
time-filtering the stored data; and
managing memory of the storage device (230) to prepare the filtered data for display of the visual image.

15. The apparatus (100) according to any one of claims 12 to 14, wherein the predetermined timing sequence further defines a start time and an end time for a secondary processing step, wherein the start time for the secondary processing step occurs substantially immediately after the end time of one of the primary processing steps, and
wherein the processing module (220) is further configured to perform the secondary processing step according to the predetermined timing sequence.

16. The apparatus (100) according to claim 15, wherein the start time for the secondary processing step occurs substantially immediately after the end time of a final primary processing step for such frame.

17. The apparatus (100) according to claim 15 or claim 16, wherein the processing module (220) is further configured to perform the secondary processing step on a predecessor frame of data to such frame on which the plurality of first processing steps is performed.

18. The apparatus (100) according to any one of claims 15 to 17, further comprising a storage device (230) accessible by the processing module and a display device (560) accessible by the processing module (220) and wherein the secondary processing step includes displaying the visual image represented by the filtered data of a predecessor frame on the display device (560).

19. The apparatus (100) according to any one of claims 15 to 18, wherein the secondary processing step further includes converting the filtered data of the predecessor frame from a first coordinate system to a second coordinate system.

20. The apparatus (100) according to any one of claims 12 to 19, wherein the acquisition module (122,124,126,128) is further configured to acquire the frames at regularly spaced time intervals.

21. The apparatus (100) according to any one of claims 12 to 20, wherein the processing module (220) is further configured to initiate performance of the plurality of primary processing steps in response to a frame index interrupt signal received from the instrument.

22. The apparatus (100) according to claim 12 further comprising:
a storage device (230); and
a display device (560); and wherein
the acquisition module (122,124,126,128) is configured to acquire a plurality of frames of data from the instrument (130,132,134,136) at regularly spaced time intervals; and
the processing module (220) is configured to respond to a frame index interrupt signal received from the instrument (130,132,134,136) by sequentially performing a plurality of primary processing steps on each such frame of image data acquired by the acquisition module (122,124,126,128) according to a predetermined timing sequence in which:
such frame of image data is stored on the storage device (230);
substantially immediately thereafter, the stored data are time-filtered;
substantially immediately thereafter, management activity is performed on memory of the storage device (230) to prepare the filtered data for display;
substantially immediately thereafter, the filtered data of a predecessor frame are converted from a first coordinate system to a second coordinate system; and
substantially immediately thereafter, the image represented by the converted data of the predecessor frame is displayed on the display device (560).

## Patentansprüche

1. Verfahren zur Echtzeit-synchronisierten Verarbeitung von Instrumenten(130, 132, 134, 136)-Daten, mit den Schritten:
- Gewinnen einer Mehrzahl von Datenblöcken von einem Instrument, und
- sequentielles Durchführen einer Mehrzahl von ersten Verarbeitungsschritten an den Daten eines derartigen Blocks für jeden derartigen Block gemäß einer vorbestimmten Synchronisierungssequenz, die eine Startzeit und eine Endzeit für jeden ersten Verarbeitungsschritt bestimmt, wobei die Startzeit eines jeden derartigen ersten Verarbeitungsschritts im wesentlichen unmittelbar nach der Endzeit eines vorangehenden ersten Verarbeitungsschritts eintritt.

2. Verfahren nach Anspruch 1, bei dem jeder Datenblock ein visuell wahrnehmbares Bild repräsentiert.

3. Verfahren nach Anspruch 2, bei dem die Mehrzahl von ersten Verarbeitungsschritten umfasst:
- Speichern eines derartigen Datenblocks in einer Speichereinrichtung (230),
- Filtern der gespeicherten Daten mit einem Zeitfilter, und
- Organisieren des Speichers der Speichereinrichtung (230) zur Vorbereitung der gefilterten Daten für eine Anzeige des visuell wahrnehmbaren Bildes.

4. Verfahren nach einem der Ansprüche 1 bis 3, bei dem die vorbestimmte Synchronisierungssequenz ferner eine Startzeit und eine Endzeit für einen zweiten Verarbeitungsschritt bestimmt, wobei die Startzeit für den zweiten Verarbeitungsschritt im wesentlichen unmittelbar nach der Endzeit eines der ersten Verarbeitungsschritte eintritt, und wobei das Verfahren ferner das Durchführen des zweiten Verarbeitungsschritts gemäß der vorbestimmten Synchronisierungssequenz umfasst.

5. Verfahren nach Anspruch 4, bei dem die Startzeit für den zweiten Verarbeitungsschritt im wesentlichen unmittelbar nach der Endzeit eines letzten ersten Verarbeitungsschritts für einen derartigen Block eintritt.

6. Verfahren nach Anspruch 4 oder Anspruch 5, bei dem der zweite Verarbeitungsschritt an einem Datenblock durchgeführt wird, der einem solchen Block vorangeht, an dem die Mehrzahl von ersten Verarbeitungsschritten durchgeführt wird.

7. Verfahren nach einem der Ansprüche 4 bis 6, bei dem der zweite Verarbeitungsschritt die Wiedergabe des visuell wahrnehmbaren Bildes, das durch die gefilterten Daten eines vorangehenden Blocks repräsentiert wird, auf einer Anzeigeeinrichtung (560) umfasst.

8. Verfahren nach einem der Ansprüche 4 bis 7, bei dem der zweite Verarbeitungsschritt ferner das Umwandeln der gefilterten Daten des vorangehenden Blocks von einem ersten Koordinatensystem in ein zweites Koordinatensystem umfasst.

9. Verfahren nach einem der vorhergehenden Ansprüche, bei dem die Blöcke in gleichmäßig voneinander beabstandeten Zeitintervallen gewonnen werden.

10. Verfahren nach einem der vorhergehenden Ansprüche, bei dem der Schritt des sequentiellen Durchführens einer Mehrzahl von ersten Verarbeitungsschritten durch ein Blockindex-Unterbrechersignal eingeleitet wird.

11. Verfahren nach Anspruch 1 mit den Schritten:
- Gewinnen einer Mehrzahl von Bilddatenblöcken von einem Instrument (130, 132, 134, 136) in gleichmäßig voneinander beabstandeten Zeitintervallen, und
- für jeden derartigen Block in Reaktion auf ein Blockindex-Unterbrechersignal und gemäß einer vorbestimmten Synchronisierungssequenz:
-- Speichern des Bilddatenblocks in einer Speichereinrichtung (230),
-- im wesentlichen unmittelbar danach Filtern der gespeicherten Daten mit einem Zeitfilter,
-- im wesentlichen unmittelbar danach Organisieren des Speichers der Speichereinrichtung (230) zur Vorbereitung der gefilterten Daten zur Wiedergabe,
-- im wesentlichen unmittelbar danach Umwandeln der gefilterten Daten eines vorangehenden Blocks aus einem ersten Koordinatensystem in ein zweites Koordinatensystem, und
-- im wesentlichen unmittelbar danach Wiedergeben eines Bildes, welches durch die umgewandelten Daten des vorangehenden Blocks repräsentiert wird, auf einer Anzeigeneinrichtung (560).

12. Vorrichtung (100) für die Echtzeit-synchronisierte Verarbeitung von Daten von einem Instrument (130, 132, 134, 136) mit:
- einem Gewinnungsmodul (122, 124, 126, 128), das dazu eingerichtet ist, eine Mehrzahl von Datenblöcken von dem Instrument zu gewinnen, und
- einem Verarbeitungsmodul (220), das dazu eingerichtet ist, sequentiell eine Mehrzahl von ersten Verarbeitungsschritten an jedem derartigen, von dem Gewinnungsmodul (122, 124, 126, 128) gewonnenen Datenblock gemäß einer vorbestimmten Synchronisierungssequenz' durchzuführen, welche eine Startzeit und eine Endzeit für jeden ersten Verarbeitungsschritt bestimmt, wobei die Startzeit eines jeden derartigen ersten Verarbeitungsschritts im wesentlichen unmittelbar nach der Endzeit eines vorangehenden ersten Verarbeitungsschritts eintritt.

13. Vorrichtung (100) nach Anspruch 12, bei dem jeder Datenblock ein visuell wahrnehmbares Bild repräsentiert.

14. Vorrichtung (100) nach Anspruch 13, die ferner eine durch das Verarbeitungsmodul (220) zugängliche Speichereinrichtung (230) umfasst, und bei der die Mehrzahl von ersten Verarbeitungsschritten umfasst:
- Speichern eines derartigen Datenblocks in der Speichereinrichtung (230),
- Zeitfiltern der gespeicherten Daten, und
- Organisieren des Speichers der Speichereinrichtung (230) zur Vorbereitung der gefilterten Daten für die Wiedergabe des visuell wahrnehmbaren Bildes.

15. Vorrichtung (100) nach einem der Ansprüche 12 bis 14, bei dem die vorbestimmte Synchronisierungssequenz ferner eine Startzeit und eine Endzeit für einen zweiten Verarbeitungsschritt bestimmt, wobei die Startzeit für den zweiten Verarbeitungsschritt im wesentlichen unmittelbar nach der Endzeit eines der ersten Verarbeitungsschritte eintritt, und wobei das Verarbeitungsmodul (220) ferner dazu eingerichtet ist, den zweiten Verarbeitungsschritt gemäß der vorbestimmten Synchronisierungssequenz durchzuführen.

16. Vorrichtung (100) nach Anspruch 15, bei dem die Startzeit für den zweiten Verarbeitungsschritt im wesentlichen unmittelbar nach der Endzeit eines letzten ersten Verarbeitungsschritts für einen derartigen Block eintritt.

17. Vorrichtung (100) nach Anspruch 15 oder Anspruch 16, bei dem das Verarbeitungsmodul (220) ferner dazu eingerichtet ist, den zweiten Verarbeitungsschritt an einem Datenblock durchzuführen, der einem solchen Block vorangeht, an dem die Mehrzahl von ersten Verarbeitungsschritten durchgeführt wird.

18. Vorrichtung (100) nach einem der Ansprüche 15 bis 17, die ferner eine durch das Verarbeitungsmodul zugängliche Speichereinrichtung (230) und eine durch das Verarbeitungsmodul (220) zugängliche Anzeigeeinrichtung (560) umfasst, wobei der zweite Verarbeitungsschritt das Wiedergeben des visuell wahrnehmbaren Bildes, das durch die gefilterten Daten eines vorangehenden Blocks repräsentiert wird, auf der Anzeigeneinrichtung (560) umfasst.

19. Vorrichtung (100) nach einem der Ansprüche 15 bis 18, bei dem der zweite Verarbeitungsschritt ferner das Umwandeln der gefilterten Daten des vorangehenden Blocks von einem ersten Koordinatensystem in ein zweites Koordinatensystem umfasst.

20. Vorrichtung (100) nach einem der Ansprüche 12 bis 19, bei dem das Gewinnungsmodul (122, 124, 126, 128) ferner dazu eingerichtet ist, die Blöcke in gleichmäßig voneinander beabstandeten Zeitintervallen zu gewinnen.

21. Vorrichtung (100) nach einem der Ansprüche 12 bis 20, bei dem das Verarbeitungsmodul (220) ferner dazu eingerichtet ist, die Durchführung der Mehrzahl von ersten Verarbeitungsschritten in Reaktion auf ein von dem Instrument empfangenes Blockindex-Unterbrechersignal einzuleiten.

22. Vorrichtung (100) nach Anspruch 12, die ferner umfasst:
- eine Speichereinrichtung (230), und
- eine Anzeigeeinrichtung (560), und wobei das Gewinnungsmodul (122, 124, 126, 128) dazu eingerichtet ist, eine Mehrzahl von Datenblöcken von dem Instrument (130, 132, 134, 136) in gleichmäßig voneinander beabstandeten Zeitintervallen zu gewinnen, und das Verarbeitungsmodul (220) dazu eingerichtet ist, auf ein von dem Instrument (130, 132, 134, 136) empfangenes Blockindex-Unterbrechersignal mit der sequentiellen Durchführung einer Mehrzahl von ersten Verarbeitungsschritten an jedem derartigen, von dem Gewinnungsmodul (122, 124, 126, 128) gewonnenen Bilddatenblock gemäß einer vorbestimmten Synchronisierungssequenz zu reagieren, in der:
-- ein derartiger Bilddatenblock in der Speichereinrichtung (230) gespeichert wird,
-- die gespeicherten Daten im wesentlichen unmittelbar danach zeitgefiltert werden,
-- im wesentlichen unmittelbar danach eine Organisationshandlung im Speicher der Speichereinrichtung (230) zur Vorbereitung der gefilterten Daten für die Anzeige durchgeführt wird,
-- im wesentlichen unmittelbar danach die gefilterten Daten eines vorangehenden Blocks von einem ersten Koordinatensystem in ein zweites Koordinatensystem umgewandelt werden, und
-- im wesentlichen unmittelbar danach das durch die umgewandelten Daten des vorangehenden Blocks repräsentierte Bild auf der Anzeigeeinrichtung (560) angezeigt wird.

## Revendications

1. Un procédé pour le traitement synchronisé en temps réel de données d'instrument (130, 132, 134, 136), comprenant les étapes suivantes :
on acquiert une multiplicité de trames de données à partir d'un instrument; et
pour chacune de ces trames, on effectue séquentiellement une multiplicité d'étapes de traitement principales sur les données d'une telle trame, conformément à une séquence temporelle prédéterminée qui définit un instant de début et un instant de fin pour chaque étape de traitement principale,
dans lequel l'instant de début de chaque étape de traitement principale apparaît pratiquement immédiatement après l'instant de fin d'une étape de traitement principale précédente.

2. Le procédé selon la revendication 1, dans lequel chaque trame de données représente une image visuelle.

3. Le procédé selon la revendication 2, dans lequel la multiplicité d'étapes de traitement principales comprend :
le stockage d'une telle trame de données dans un dispositif de stockage (230);
le filtrage des données stockées avec un filtre temporel; et
la gestion de mémoire du dispositif de stockage (230) pour préparer les données filtrées pour la visualisation de l'image visuelle.

4. Le procédé selon l'une quelconque des revendications 1 à 3,
dans lequel la séquence temporelle prédéterminée définit en outre un instant de début et un instant de fin pour une étape de traitement secondaire, dans lequel l'instant de début pour l'étape de traitement secondaire apparaît pratiquement immédiatement après l'instant de fin de l'une des étapes de traitement principales, et
dans lequel le procédé comprend en outre l'accomplissement de l'étape de traitement secondaire conformément à la séquence temporelle prédéterminée.

5. Le procédé selon la revendication 4, dans lequel l'instant de début pour l'étape de traitement secondaire apparaît pratiquement immédiatement après l'instant de fin d'une étape de traitement principale finale pour une telle trame.

6. Le procédé selon la revendication 4 ou la revendication 5,
dans lequel l'étape de traitement secondaire est effectuée sur une trame de données antérieure à une trame sur laquelle la multiplicité de premières étapes de traitement est effectuée.

7. Le procédé selon l'une quelconque des revendications 4 à 6,
dans lequel l'étape de traitement secondaire comprend la visualisation sur un dispositif de visualisation (560) de l'image visuelle représentée par les données filtrées d'une trame antérieure.

8. Le procédé selon l'une quelconque des revendications 4 à 7,
dans lequel l'étape de traitement secondaire comprend en outre la conversion des données filtrées de la trame antérieure d'un premier système de coordonnées à un second système de coordonnées.

9. Le procédé selon l'une quelconque des revendications précédentes, dans lequel les trames sont acquises à des intervalles de temps régulièrement espacés.

10. Le procédé selon l'une quelconque des revendications précédentes, dans lequel l'étape consistant à effectuer séquentiellement une multiplicité de premières étapes de traitement est déclenchée par un signal d'interruption d'index de trame.

11. Le procédé selon la revendication 1, comprenant les étapes suivantes :
on acquiert une multiplicité de trames des données d'image à partir d'un instrument (130, 132, 134, 136) à des intervalles de temps régulièrement espacés; et
pour chacune de ces trames, en réponse à un signal d'interruption d'index de trame et conformément à une séquence temporelle prédéterminée :
on stocke la trame de données d'image dans un dispositif de stockage (230);
pratiquement immédiatement à la suite, on filtre les données stockées avec un filtre temporel;
pratiquement immédiatement à la suite, on gère la mémoire du dispositif de stockage (230) pour préparer les données filtrées pour la visualisation;
pratiquement immédiatement à la suite, on convertit les données filtrées d'un trame antérieure d'un premier système de coordonnées à un second système de coordonnées; et
pratiquement immédiatement à la suite, on visualise sur un dispositif de visualisation (560) une image représentée par les données converties de la trame antérieure.

12. Un appareil (100) pour le traitement synchronisé en temps réel de données provenant d'un instrument (130, 132, 134, 136) comprenant :
un module d'acquisition (122, 124, 126, 128) configuré pour acquérir une multiplicité de trames de données à partir de l'instrument; et
un module de traitement (220) configuré pour accomplir séquentiellement une multiplicité d'étapes de traitement principales sur chaque trame de données acquise par le module d'acquisition (122, 124, 126, 128) conformément à une séquence temporelle prédéterminée qui définit un instant de début et un instant de fin pour chaque étape de traitement principale,
dans lequel l'instant de début de chaque étape de traitement principale apparaît pratiquement immédiatement après l'instant de fin d'une étape de traitement principale précédente.

13. L'appareil (100) selon la revendication 12, dans lequel chaque trame de données représente une image visuelle.

14. L'appareil (100) selon la revendication 13, comprenant en outre un dispositif de stockage (230) accessible par le module de traitement (220), et dans lequel la multiplicité d'étapes de traitement principales comprend :
le stockage d'une telle trame de données dans le dispositif de stockage (230);
le filtrage temporel des données stockées; et
la gestion de mémoire du dispositif de stockage (230) pour préparer les données filtrées pour la visualisation de l'image visuelle.

15. L'appareil (100) selon l'une quelconque des revendications 12 à 14, dans lequel la séquence temporelle prédéterminée définit en outre un instant de début et un instant de fin pour une étape de traitement secondaire, dans lequel l'instant de début pour l'étape de traitement secondaire apparaît pratiquement immédiatement après l'instant de fin de l'une des étapes de traitement principales, et
dans lequel le module de traitement (220) est en outre configuré pour accomplir l'étape de traitement secondaire conformément à la séquence temporelle prédéterminée.

16. L'appareil (100) selon la revendication 15, dans lequel l'instant de début pour l'étape de traitement secondaire apparaît pratiquement immédiatement après l'instant de fin d'une étape de traitement principale finale pour une telle trame.

17. L'appareil (100) selon la revendication 15 ou la revendication 16, dans lequel le module de traitement (220) est en outre configuré pour accomplir l'étape de traitement secondaire sur une trame de données antérieure à une trame sur laquelle la multiplicité de premières étapes de traitement est effectuée.

18. L'appareil (100) selon l'une quelconque des revendications 15 à 17, comprenant en outre un dispositif de stockage (230) accessible par le module de traitement et un dispositif de visualisation (560) accessible par le module de traitement (220), et dans lequel l'étape de traitement secondaire comprend la visualisation sur le dispositif de visualisation (560) de l'image visuelle représentée par les données filtrées d'une trame antérieure.

19. L'appareil (100) selon l'une quelconque des revendications 15 à 18, dans lequel l'étape de traitement secondaire comprend en outre la conversion des données filtrées de la trame antérieure d'un premier système de coordonnées à un second système de coordonnées.

20. L'appareil (100) selon l'une quelconque des revendications 12 à 19, dans lequel le module d'acquisition (122, 124, 126, 128) est en outre configuré pour acquérir les trames à des intervalles de temps régulièrement espacés.

21. L'appareil (100) selon l'une quelconque des revendications 12 à 20, dans lequel le module de traitement (220) est en outre configuré pour déclencher l'accomplissement de la multiplicité d'étapes de traitement principales en réponse à un signal d'interruption d'index de trame reçu à partir de l'instrument.

22. L'appareil (100) selon la revendication 12, comprenant en outre :
un dispositif de stockage (230); et
un dispositif de visualisation (560); et dans lequel
le module d'acquisition (122, 124, 126, 128) est configuré pour acquérir une multiplicité de trames de données à partir de l'instrument (130, 132, 134, 136), à des intervalles de temps régulièrement espacés; et
le module de traitement (220) est configuré pour réagir à un signal d'interruption d'index de trame reçu à partir de l'instrument (130, 132, 134, 136) en accomplissant séquentiellement une multiplicité d'étapes de traitement principales sur chaque trame de données d'image acquise par le module d'acquisition (122, 124, 126, 128) conformément à une séquence temporelle prédéterminée, dans laquelle :
une telle trame de données d'image est stockée dans le dispositif de stockage (230);
pratiquement immédiatement à la suite, les données stockées sont soumises à un filtrage temporel;
pratiquement immédiatement à la suite, une activité de gestion est effectuée sur la mémoire du dispositif de stockage (230) pour préparer les données filtrées pour la visualisation;
pratiquement immédiatement à la suite, les données filtrées d'un trame antérieure sont converties d'un premier système de coordonnées à un second systèmé de coordonnées; et
pratiquement immédiatement à la suite, l'image représentée par les données converties de la trame antérieure est visualisée sur le dispositif de visualisation (560).
